# EUROPÄISCHE PATENTSCHRIFT

(11) **EP 0 586 346 B1**
(45) Veröffentlichungstag und Bekanntmachung des Hinweises auf die Patenterteilung: **17.04.1996**
(21) Anmeldenummer: 93810609.3
(22) Anmeldetag: 25.08.1993
(51) Int. Cl.: C07C 303/32, C07C 309/32, D06L 3/12, C11D 3/42

(54) **Verfahren zur Herstellung von Distyrylbiphenylverbindungen**
Method for the preparation of distyrylbiphenyl compounds
Procédé pour la préparation des composés distyrylbiphényliques

(30) Priorität: 03.09.1992 CH 2764/92
(43) Veröffentlichungstag der Anmeldung: 09.03.1994
(73) Patentinhaber: CIBA-GEIGY AG, CH-4002 Basel (CH)
(72) Erfinder: Guglielmetti, Leonardo, Dr., CH-4103 Bottmingen (CH)

(56) Entgegenhaltungen:
- EP-A- 0 298 361
- EP-A- 0 364 403
- DE-A- 1 793 482

## Beschreibung

Die Anmeldung betrifft ein neues Verfahren zur Herstellung von Distyrylbiphenylverbindungen.

Verfahren zur Herstellung von Distyrylbiphenylverbindungen über die Wittig-Horner Reaktion sind z.B. aus der DE-A-1793482 allgemein bekannt. Von den dort vorgeschlagenen Reaktionslösungsmitteln haben sich in der Praxis nur Dimethylformamid und Dimethylsulfoxid bewährt (EP-A-364403) und von den vorgeschlagenen Basen findet heute im allgemeinen nur Natriummethylat Verwendung.

Die Verwendung von Dimethylformamid als Reaktionslösungsmittel ist aber mit grossen Nachteilen verbunden. So ist z.B. Dimethylformamid in Gegenwart von Basen bekanntlich instabil. Unter den in der Praxis verwendeten Reaktionsbedingungen wird ein kleiner Teil des Dimethylformamids durch Natriummethylat verseift. Das dabei entstehende Dimethylamin verursacht nicht nur ökologische Probleme, sondern verleiht auch der isolierten Distyrylbiphenyl-Verbindung einen unangenehmen, schwer entfernbaren Dimethylamin-Geruch. Ferner ist die verwendete Base (Natriummethylat) in Dimethylformamid schlecht löslich, so dass in der Praxis die Base als eine 30 %ige methanolische Natriummethylat-Lösung eingesetzt wird. Diese Arbeitsweise ist mit dem Nachteil verbunden, dass zwei Lösungsmittel (Dimethylformamid und Methanol) regeneriert werden müssen. Als aprotisches dipolares Lösungsmittel mit hohem Siedepunkt (154°C) kann Dimethylformamid in der Praxis deshalb nur mit einem Verlust von 5 bis 10 % regeneriert werden.

Die Verwendung von Dimethylsulfoxid als Reaktionslösungsmittel ist mit ähnlichen Nachteilen verbunden. Dimethylsulfoxid ist zwar im Gegensatz zu Dimethylformamid in Gegenwart von Basen stabil und die verwendete Base (Natriummethylat) ist in Dimethylsulfoxid gut löslich. Dimethylsulfoxid ist jedoch nicht stabil gegenüber Oxidations-Reduktions-Reaktionen, wobei Dimethylsulfid, Dimethyldisulfid und besonders das sehr unangenehm riechende Methylmercaptan entsteht. Ferner hat Dimethylsulfoxid einen Siedepunkt von 189°C und kann deshalb in der Praxis ebenfalls nur mit einem Verlust von 5 bis 10 % regeneriert werden, wobei in diesem Fall die Verluste, wegen des im Vergleich zu Dimethylformamid doppelt so hohen Preises, bedeutender sind.

Die Durchführung der obengenannten Reaktion mit einem der oben erwähnten Lösungsmitteln und Natriummethylat als Base ist auch bei der Aufarbeitung mit grossen Nachteilen verbunden. Nach weitgehender Rückgewinnung des verwendeten Lösungsmittels muss die Kondensationsmasse, damit das Kondensationsprodukt durch Kristallisation in genügend reinem Zustand isoliert werden kann, zuerst in Wasser gelöst und dann klärfiltriert werden. Da bei Wittig-Horner Reaktionen nicht nur das gewünschte Kondensationsprodukt, sondern auch äquimolekulare Mengen des entsprechenden Salzes des Phosphorsäuredialkylesters entstehen, müssen die oben erwähnten Operationen, wegen des starken Aussalzeffektes dieses Phosphorsäuresalzes, in starker Verdünnung durchgeführt werden. Nach Isolierung der Distyrylbiphenyl-Verbindung müssen somit grosse Mengen von stark verdünnten und phosphorsäuredialkylesterhaltigen Mutterlaugen beseitigt werden.

Es wurde nun gefunden, dass man Distyrylbiphenylverbindungen der Formel (1) 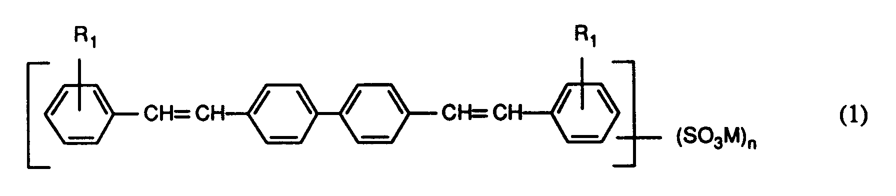
durch Kondensation einer Verbindung der Formel (2) 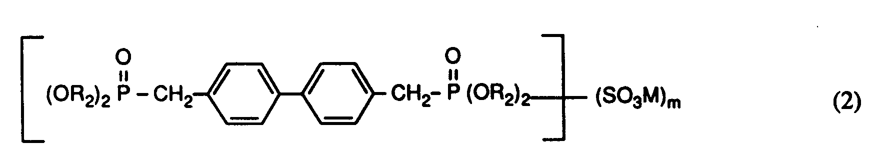
mit einer Verbindung der Formel (3) 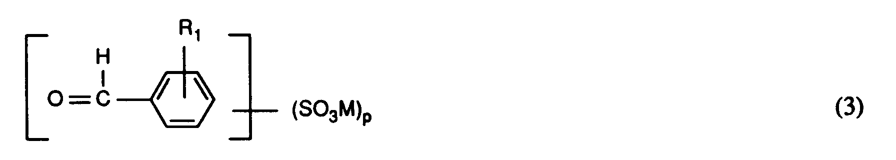
worin
R₁ Wasserstoff, C₁-C₅-Alkyl oder Halogen;
R₂ C₁-C₈-Alkyl;
M ein salzbildendes farbloses Kation;
n 2 oder 4;
m 0 oder 2; und
p 0 oder 1; unter der Voraussetzung, dass m + p 2 oder 4 ist,
bedeuten,
herstellen kann, wenn man die Kondensation in flüssigem Ammoniak und in Gegenwart von stark alkalisch reagierenden Substanzen durchführt.

Als Reste R₁ kommen z.B. Wasserstoff, Methyl, Ethyl, Propyl, Butyl, t-Butyl, Propyl, Chlor oder Brom in Frage, wobei Wasserstoff, Methyl, Ethyl und Chlor bevorzugt sind. Als Reste R₂ werden üblicherweise Methyl, Ethyl, Propyl, Butyl, Hexyl oder Octyl verwendet, wobei Methyl, Ethyl, Propyl und Butyl bevorzugt sind.

Als Kationen M kommen z.B. Alkalimetallkationen wie Natrium und Kalium, Erdalkalimetallkationen wie Kalzium und Magnesium sowie Ammoniumkationen in Betracht.

Bevorzugt werden als Kondensationspartner die Verbindungen der Formeln (4) und (5) 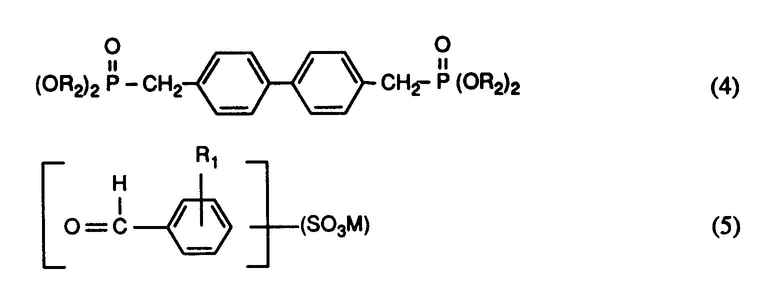
eingesetzt, worin
R₁ Wasserstoff, C₁-C₅-Alkyl oder Halogen;
R₂ C₁-C₈-Alkyl, und
M ein salzbildendes farbloses Kation
bedeutet.

In einem besonders bevorzugtem Verfahren werden die Verbindungen der Formeln (4) und (6) 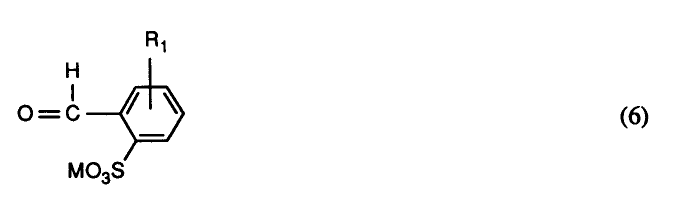
kondensiert, worin
R₁ Wasserstoff, C₁-C₅-Alkyl oder Chlor;
R₂ C₁-C₈-Alkyl; und
M ein salzbildendes farbloses Kation
bedeutet.

Die Ausgangsverbindungen der Formel (2) sind z.B. aus der DE-A-1793482 bekannt und lassen sich allgemein durch Umsetzung der 4,4'-Bis-(chlormethyl)-diphenyl-Derivate mit Alkylphosphiten, wie Trimethylphosphit, nach der Methode von Arbuzov gewinnen.

Die Verbindungen der Formel (3) lassen sich z.B. durch Umsetzung chlorierter Benzaldehyde mit Natriumsulfit in Wasser und unter Druck darstellen.

Nach dem erfindungsgemässen Verfahren lassen sich insbesonders die Verbindungen der Formeln (7)-(12) herstellen 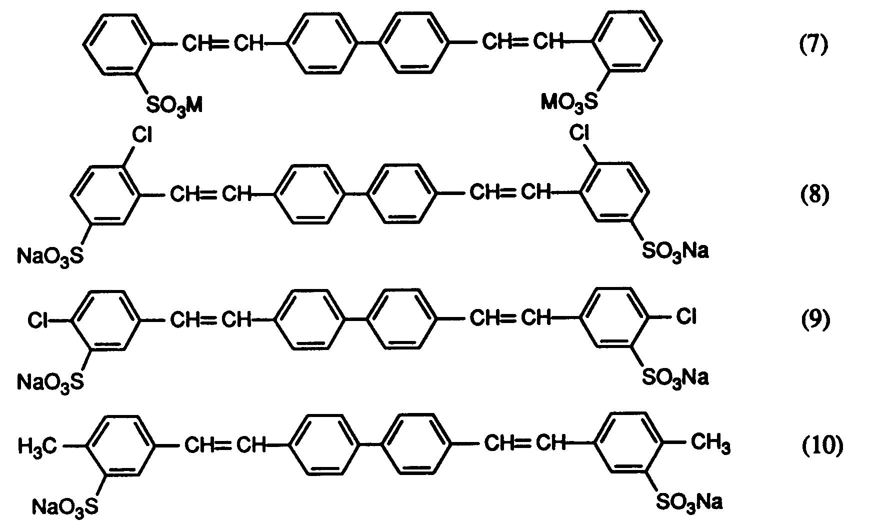 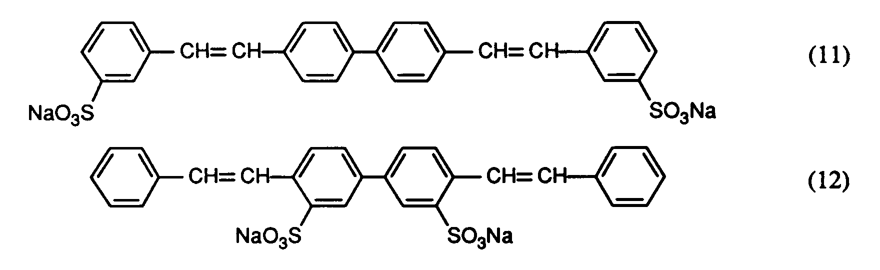
Die Kondensation wird erfindungsgemäss in flüssigem Ammoniak, z.B. bei Temperaturen zwischen -40°C und 25°C, bevorzugt zwischen 0°C und 25°C und besonders bevorzugt zwischen 10°C und 20°C, in Gegenwart von starken Basen, durchführt. Aus dem Siedepunkt des Ammoniaks von -33,35°C (760 Torr) ergibt sich, dass bei darüberliegenden Temperaturen bei Überdruck gearbeitet werden muss.

Als für die Kondensationsreaktion geeignete stark alkalisch reagierende Substanzen sind z.B. die Alkali- oder Erdalkalimetalle, wie Lithium, Natrium, Kalium, Magnesium und Calcium sowie ihre stark basischen Verbindungen, z.B. Hydroxide, Amide oder Alkoholate, sowie stark basische Ionenaustauscher geeignet. Als Alkoholate kommen im wesentlichen solche zur Anwendung, die sich von offenkettigen, verzweigten oder cyclischen niederen aliphatischen Alkoholen mit 1 bis 8 Kohlenstoffatomen, vorzugsweise 1 bis 4 Kohlenstoffatomen wie Methanol, Ethanol, Propanol, Butanol, Isopropanol und tert-Butanol ableiten. Diese Alkoholate werden vorzugsweise in Form der entsprechenden alkoholischen Lösung eingesetzt. Vor allem verwendet man als stark alkalisch reagierende Substanzen Alkalimetalle oder deren stark basische Verbindungen, vorzugsweise Amide, Hydride oder Alkoholate von Alkalimetallen oder Gemische davon, insbesondere Alkoholate oder Amide des Natriums und vor allem Natriumamid.

Bevorzugt werden Natrium- oder Kaliumverbindungen verwendet, wobei deren Hydroxide, Alkoholate und Amide von praktischer Bedeutung sind. Von besonderer Bedeutung ist die Verwendung von frisch hergestelltem Natriumamid. Dazu wird z.B. Natrium in das vorgelegte flüssige Ammoniak, vorzugsweise in Gegenwart eines geeigneten Katalysators, wie Eisen(II)-chlorid oder Eisen(II)-nitrat, gegeben.

Die genannten stark alkalisch reagierenden Substanzen werden bevorzugt im wasserfreien Zustand, entweder für sich oder als Gemisch, verwendet. Kleine Mengen Wasser, so wie sie in manchen technischen starken Basen vorkommen, stören die Kondensation jedoch nicht. Die starken Basen weisen stark unterschiedliche Löslichkeiten in flüssigem Ammoniak auf. Amide sind z.B. in flüssigem Ammoniak sehr gut löslich, während Hydroxide nur geringe Löslichkeiten aufweisen. Je nach Art der verwendeten Base ist es manchmal von Vorteil, kleine Mengen eines protischen Lösungsmittels als Hilfslösungsmittel zu verwenden. Als protische Lösungsmittel kommen Wasser oder bevorzugt offenkettige, verzweigte oder cyclische niedermolekulare aliphatische Alkohole mit 1 bis 8 Kohlenstoffatomen zur Anwendung. Von besonderer praktischen Bedeutung ist jedoch die Verwendung von Methanol als Hilfslösungsmittel, da die meisten der verwendeten Hydroxide in Methanol sehr gut löslich sind.

Die einzusetzende Basenmenge kann sich in weiten Grenzen bewegen. Bevorzugt werden aber mindestens zwei bis drei Äquivalente Base pro Äquivalent einer Verbindung der Formel (2) verwendet. Zur Durchführung der Kondensation kann die Base während oder nach der Zudosierung der Verbindung der Formel (3), zu der im Reaktionskessel vorgelegten Verbindung der Formel (2), zudosiert werden. Bevorzugt wird die Base zu den im Reaktionskessel vorgelegten Verbindungen der Formeln (2) und (3) zudosiert.

Ein Überschuss an Base kann nach der Kondensationsreaktion durch Zugabe von sauren Verbindungen neutralisiert werden. Beispiele für saure Verbindungen sind z.B. Chlorwasserstoff, Schwefelsäure und Ammoniumchlorid.

Das Verhältnis der Edukte der Formeln (2) und (3) liegt dabei bevorzugt bei 1:2 bis 1:2,5 und besonders bevorzugt bei 1:2,1 bis 1:2,2.

Ein besonderer Vorteil des erfindungsgemässen Verfahrens liegt in der leichten Abtrennbarkeit des Produktes von den Nebenprodukten. So liegt das Kondensationsprodukt der Formel (1) als unlöslicher Bestandteil vor und kann durch Filtration abgetrennt werden. Der als Nebenprodukt entstehende Phosphatester, sowie die weiteren Nebenprodukte der Reaktion, bleiben dagegen im flüssigen Ammoniak gelöst. Das als Lösungsmittel verwendete flüssige Ammoniak kann dann durch Verdampfen und erneutes Kondensieren von allen Verunreinigungen gereinigt und wiederverwendet werden.

Die so erhaltenen Distyrylbiphenylverbindungen werden üblicherweise zum optischen Aufhellen von Textilmaterial wie Baumwolle, Polyamid und Wolle oder zum optischen Aufhellen von Papier verwendet. Sie können dazu in flüssige und feste Waschmittel, Applikationsflotten oder Streichmassen eingearbeitet werden.

Sie werden dazu im allgemeinen auf die für die jeweilige Anwendung optimale Konzentration durch den Zusatz von weiteren Hilfsmitteln oder Wasser verdünnt.

Die so erhaltenen Formulierungen können zusätzlich die üblichen Formulierungshilfsmittel wie Dispergatoren, Gerüstbildner, Schutzkolloide, Stabilisatoren, Konservierungsmittel, Parfümierungsmittel, Pigmente, Enzyme sowie Sequestriermittel enthalten.

Als Dispergatoren werden bevorzugt nichtionische, wie z.B. Fettalkohole, Ethoxylierungs-produkte von Fettalkoholen oder Fettsäuren, oder anionische, wie die Kondensationsprodukte von aromatischen Sulfonsäuren mit Formaldehyd so z.B. solche auf der Basis von Ditolylethersulfonsäuren oder Naphthalinsulfonaten, oder Ligninsulfonate, eingesetzt.

Als Gerüstbildner oder Schutzkolloide kommen z.B. modifizierte Polysaccharide die sich von der Zellulose ableiten oder Heteropolysaccharide wie Xanthan, Carboxymethylcellulose sowie Aluminium- oder Magnesiumsilikate in Frage.

Als weitere Hilfsmittel zur Stabilisierung können z.B. Ethylenglykol, Propylenglykol sowie weitere Dispergiermittel zugesetzt werden.

Als Konservierungsmittel finden z.B. Verbindungen wie 1,2-Benzisothiazolin-3-on, Formaldehyd oder Chloracetamid Verwendung.

Die folgenden Beispiele erläutern die Erfindung, ohne sie darauf zu beschränken.

### Beispiel 1:

Eine in Serie aufgestellte Apparatur besteht in der angegebenen Reihenfolge aus:
- Einem ersten 1 l BUECHI®-Glasautoklaven, ausgerüstet mit Kühl/Heiz-Mantel, Manometer (0 bis 10 bar) und TESCOM®-Back-Pressure-Regulator (0 bis 7 bar), Rührer mit Permanent-Magnetantrieb, Thermometerhülse, Einlaufstutzen für flüssiges Ammoniak und Natrium sowie einer Berstscheibe (10 bar),
- einem zweiten 1,5 l BUECHI®-Glasautoklaven ausgerüstet mit Kühl/Heiz-Mantel, Manometer (0 bis 10 bar) und TESCOM®-Back-Pressure-Regulator (0 bis 7 bar), Rührer mit Permanent-Magnetantrieb, Thermometerhülse, Einlaufstutzen für flüssiges Ammoniak und Natriumamid-Ammoniak-Suspension, Boden-Auslaufventil und einer Berstscheibe (10 bar) sowie
- einer 2 l LIGACON®-Hochdruckautoklavennutsche, ausgerüstet mit Kühl/Heiz-Mantel, Manometer (0 bis 10 bar) und TESCOM®-Back-Pressure-Regulator (0 bis 7 bar), Rührer mit Permanent-Magnetantrieb, Thermometerhülse, Einlaufstutzen für die Reaktionsprodukt-Suspension, Sintermetallplatte mit einer Porengrösse von 10 Micron und einem mit Stoff überdeckten SEITZ®-Filter Ko 0, Boden-Auslaufventil mit Verbindung zum zweiten Glasautoklav und einer Berstscheibe 10 bar,
Diese Apparatur wird wie folgt bedient:
Im ersten Glasautoklaven werden 130 g flüssiges Ammoniak bei -10°C (4,2 bar) vorgelegt. Der Autoklav wird auf -40°C abgekühlt und auf atmosphärischen Druck entlastet. Bei dieser Temperatur werden nun unter Rühren, nach Zugabe von einem Stück von etwa 0,5 g Natrium und von 0,5 g Eisen(III)-nitrat Nonahydrat, innerhalb von 20 Minuten insgesamt 15,5 g (0,676 Mol) Natrium in kleinen Stücken zugegeben, wobei aus dem Reaktionsgemisch ein Wasserstoffstrom entweicht. Der Autoklav wird etwa 10 Minuten nach dem Ende der Natrium-Zugabe geschlossen und die entstandene grau-schwarze Natriumamid-Suspension bei 11°C (5,8 bar) für 30 Minuten weitergerührt.

Im zweiten Glasautoklaven werden bei atmosphärischem Druck 105,7 g 4,4'-Bis-(dimethoxyphosphonomethyl)-diphenyl (98 % Aktivsubstanz; 0,26 Mol) und 148,8 g Benzaldehyd-2-sulfonsäure Natriumsalz (80 % Aktivsubstanz; 0,572 Mol) vorgelegt. Der Autoklav wird geschlossen, auf 12°C gekühlt und bei dieser Temperatur werden innerhalb von 5 Minuten 210 g flüssiges Ammoniak unter Rühren zudosiert, wobei eine blassgelbe Suspension entsteht. Diese Suspension wird auf 6°C (4,3 bar) gekühlt und unter Rühren wird innerhalb von 10 Minuten die Natriumamid-Suspension aus dem ersten Glasautoklaven zudosiert, wobei die Reaktionstemperatur von 6°C auf 14°C steigt und eine gelbe, kristalline Suspension des Reaktionsproduktes entsteht.

Der erste Autoklav wird zweimal mit je 50 g flüssigem Ammoniak gespült, die Spüllösungen in den zweiten Glasautoklaven zudosiert und das Reaktionsgemisch schliesslich für eine Stunde bei 11°C (5,8 bar) gerührt. Der Überschuss an Natriumamid wird danach durch Zugabe von 6 g (0,156 Mol) gasförmigem Chlorwasserstoff neutralisiert, das Reaktionsgemisch auf 6°C abgekühlt und bei 6°C in die Hochdruckautoklavennutsche eingeführt. Der zweite Glasautoklav wird zweimal mit je 50 g flüssigem Ammoniak gespült, wobei die Spüllösungen in die Hochdruckautoklavennutsche zudosiert werden.

Das Reaktionsgemisch wird zuerst homogen gerührt und dann ohne Rühren bei 6°C (5,6 bar) mit einem Überdruck von 1,5 bar abgenutscht, wobei das Filtrat in den zweiten Glasautoklaven eingeleitet wird. Das Nutschgut wird bei 6°C zweimal in je 100 g flüssigem Ammoniak unter Rühren aufgeschlämmt und ohne Rühren abgenutscht, wobei die Filtrate in den zweiten Autoklaven eingeleitet werden.

Der Druck in der Hochdruckautoklavennutsche und im zweiten Glasautoklav wird durch partielle Verdampfung des Ammoniaks auf atmosphärischem Druck gesenkt und danach das Nutschgut und der Filtrat-Rückstand, durch langsames Aufheizen der zwei Autoklaven auf 20°C, weitgehend vom Ammoniak befreit. Die zwei Autoklaven werden ausgeladen und das Nutschgut sowie der Filtrat-Rückstand unter Vakuum auf 100°C erhitzt und bis zur Gewichtskonstanz getrocknet.

Man erhält 153,7 g 4,4'-Bis-(2-sulfostyryl)-diphenyl Dinatriumsalz in Form eines blassgelben kristallinen Pulvers mit einem Schmelzpunkt von über 300°C, das einen Aktivsubstanzgehalt (UV-spektralphotometrisch bestimmt) von 88,6 % aufweist. Die Ausbeute an 4,4'-Bis-(2-sulfostyryl)-diphenyl Dinatriumsalz beträgt 93,1 % der Theorie. Der Filtrat-Rückstand (123 g hellgelbes, kristallines, hygroskopisches Produkt) besteht hauptsächlich aus Phosphorsäuredimethylester Natriumsalz.

Analog zu Beispiel 1 erhält man aus den entsprechenden Ausgangsmaterialen die folgenden Distyrylverbindungen der Formeln (8)-(12):

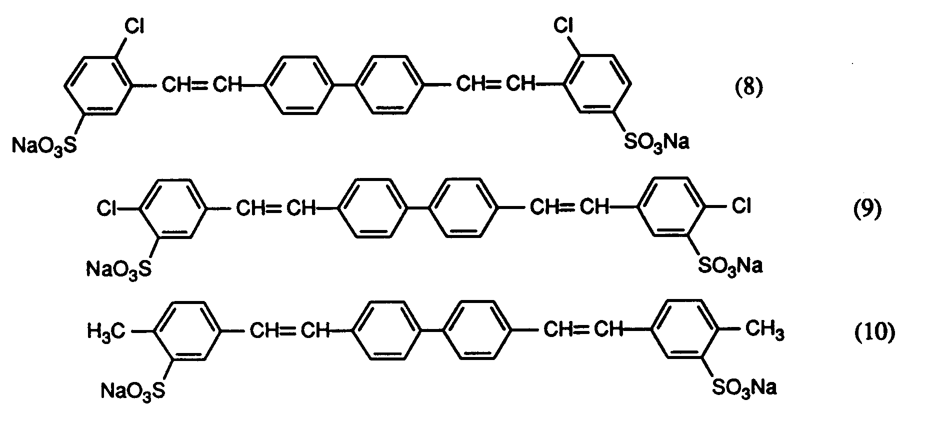

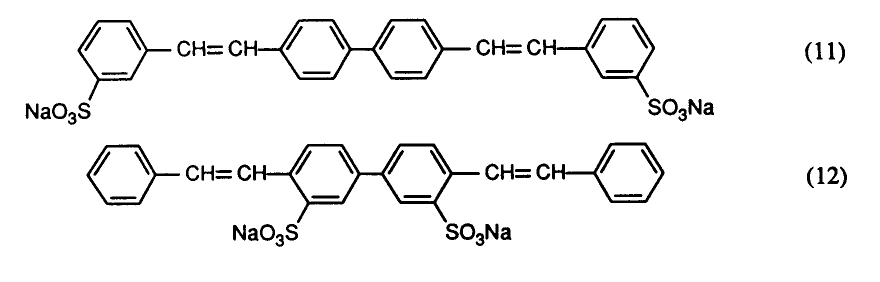

### Beispiel 2:

Beispiel 1 wird wiederholt, wobei anstelle des 10 %igen Überschusses an Benzaldehyd-2-sulfonsäure Natriumsalz nur ein 7 %iger Überschuss, d.h. 144,8 g (80 % Aktivsubstanz; 0,556 Mol) verwendet wird.

Man erhält 154,8 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 88,5 % aufweist. Die Ausbeute an 4,4'-Bis-(2-sulfostyryl)-diphenyl Dinatriumsalz beträgt 93,7 % der Theorie. Der Filtrat-Rückstand (79,1 g hellgelbes, kristallines, hygroskopisches Produkt) besteht hauptsächlich aus Phosphorsäuredimethylester Natriumsalz.

### Beispiel 3:

Beispiel 1 wird wiederholt, wobei anstelle des 10 %igen Überschusses an Benzaldehyd-2-sulfonsäure Natriumsalz nur ein 5%iger Überschuss, d.h. 142,1 g (80 % Aktivsubstanz; 0,546 Mol) verwendet wird.

Man erhält 148,8 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 89,0 % aufweist. Die Ausbeute an 4,4'-Bis-(2-sulfostyryl)-diphenyl Dinatriumsalz beträgt 90,5 % der Theorie. Der Filtrat-Rückstand (110,9 g hellgelbes, kristallines, hygroskopisches Produkt) besteht hauptsächlich aus Phosphorsäuredimethylester Natriumsalz.

### Beispiel 4:

Eine in Kaskade aufgestellte Apparatur, bestehend in der Reihenfolge aus:
- einem ersten 0,75 l Reaktionsgefäss, ausgerüstet mit Kühl/Heiz-Mantel, Rührer, Thermometer und Bodenventil,
- einem zweiten 1 l Reaktionsgefäss, ausgerüstet mit Kühl/Heiz-Mantel, Rührer, Thermometer und Bodenventil,
- einer 2 l SEITZ®-Drucknutsche, ausgerüstet mit Kühl/Heizmantel, Thermometer, Manometer und Bodenventil und
- einem dritten 0,75 l Reaktionsgefäss, ausgerüstet mit Kühl/Heiz-Mantel, Rührer, Thermometer und Bodenventil,
wird auf -40°C gekühlt.
Im ersten Reaktionsgefäss werden 140 g flüssiges Ammoniak bei -40°C vorgelegt. Bei dieser Temperatur werden nun unter Rühren, nach Zugabe von einem Stück von etwa 0,5 g Natrium und von 0,4 g Eisen(III)-nitrat Nonahydrat, innerhalb von 20 Minuten insgesamt 8,6 g (0,374 Mol) Natrium in kleinen Stücken zugegeben, wobei aus dem Reaktionsgefäss ein Wasserstoffstrom entweicht. Die entstandene grau-schwarze Natriumamid-Suspension wird bei -40°C für 30 Minuten weitergerührt.

Im zweiten Reaktionsgefäss werden in der Reihenfolge,
67,9 g 4,4'-Bis-(dimethoxyphosphonomethyl)-diphenyl (88 % Aktivsubstanz; 0,15 Mol),
89,8 g Benzaldehyd-2-sulfonsäure Natriumsalz (80 % Aktivsubstanz; 0,345 Mol) und
280 g flüssiges Ammoniak
vorgelegt und gerührt.

Zu dieser gelben Suspension wird innerhalb 5 Minuten die Natriumamid-Suspension vom ersten Reaktionsgefäss unter Rühren zudosiert, wobei die Reaktionstemperatur von -40°C auf -34°C steigt und eine rote Suspension entsteht. Das Reaktionsgemisch wird nun für 5 Stunden bei -40°C weitergerührt, wobei nach etwa 2 Stunden eine gelbe Suspension entsteht. Der Überschuss an Natriumamid wird durch Zugabe von 4 g Ammoniumchlorid (0,075 Mol) neutralisiert.

Das Reaktionsgemisch wird in die Drucknutsche eingeführt und bei -40°C mit einem Überdruck von 1,5 Bar Stickstoff durch einen mit Stoff überdeckten SEITZ®-Filter Ko 3 abgenutscht. Das Nutschgut wird zweimal mit je 100 g flüssigem Ammoniak gewaschen und durch das Durchleiten eines schwachen Stickstoffstroms, bei gleichzeitiger Temperaturerhöhung des Kühl/Heiz-Mantels von -40°C auf +26°C, weitgehend vom Ammoniak befreit.

Das Filtrat im dritten Reaktionsgefäss wird durch Erhöhen der Temperatur des Kühl/Heiz-Mantels von -40°C auf +26°C ebenfalls weitgehend vom Ammoniak befreit. Das Nutschgut und der Filtrat-Rückstand werden schliesslich im Vakuum auf 100°C erhitzt und bis zur Gewichtskonstanz getrocknet.

Man erhält 105,3 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 75,8 % aufweist. Die Ausbeute an 4,4'-Bis-(2- sulfostyryl)-diphenyl Dinatriumsalz beträgt 94,6 % der Theorie. Der Filtrat-Rückstand (57,8 g hellbraunes, kristallines, hygroskopisches Produkt) besteht hauptsächlich aus Phosphorsäuredimethylester Natriumsalz.

### Beispiel 5:

Beispiel 4 wird wiederholt, wobei anstelle des 15 %igen Überschusses an Benzaldehyd-2-sulfonsäure Natriumsalz nur ein 12 %iger Überschuss, d.h. 87,5 g (80 % Aktivsubstanz; 0,336 Mol) verwendet wird.

Man erhält 104,8 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 76,2 % aufweist. Die Ausbeute an 4,4'-Bis-(2-sulfostyryl)-diphenyl Dinatriumsalz beträgt 94,5 % der Theorie. Der Filtrat-Rückstand (53,7 g hellbraunes, kristallines, hygroskopisches Produkt) besteht hauptsächlich aus Phosphorsäuredimethylester Natriumsalz.

### Beispiel 6:

Eine in Serie aufgestellte Apparatur bestehend in der Reihenfolge aus:
- einem ersten 1 l BUECHI®-Glasautoklaven, ausgerüstet mit Kühl/Heiz-Mantel, Manometer (0 bis 10 bar) und TESCOM®-Back-Pressure-Regulator (0 bis 7 bar), Rührer mit Permanent-Magnetantrieb, Thermometerhülse, Einlaufstutzen für flüssiges Ammoniak und Natrium sowie Berstscheibe (10 bar) und
- einem zweiten 1,6 l BUECHI®-Glasautoklaven, ausgerüstet mit Kühl/Heiz-Mantel, Manometer (0 bis 10 bar) und TESCOM®-Back-Pressure-Regulator (0 bis 7 bar), Rührer mit Permanent-Magnetantrieb, Thermometerhülse, Einlaufstutzen für flüssiges Ammoniak und Natriumamid-Ammoniak-Suspension sowie Berstscheibe 10 bar,
wurde wie folgt bedient:
Im ersten Glasautoklaven werden 130 g flüssiges Ammoniak bei -10°C (4,2 bar) vorgelegt. Der Autoklav wird auf -45°C abgekühlt und auf atmosphärischen Druck entlastet. Bei dieser Temperatur werden unter Rühren, nach Zugabe von einem Stück von etwa 0,5 g Natrium und von 0,7 g Eisen(III)-nitrat Nonahydrat, innerhalb 20 Minuten insgesamt 14,4 g (0,625 Mol) Natrium in kleinen Stücken zugegeben, wobei aus dem Reaktionsgemisch einen Wasserstoffstrom entweicht. Der Autoklav wird etwa 10 Minuten nach Ende der Natrium-Zugabe geschlossen und die entstandene grau-schwarze Natriumamid-Suspension bei 12°C (6,1 bar) für 30 Minuten, weitergerührt.

Im zweiten Glasautoklaven werden unter atmosphärischem Druck 113,2 g 4,4'-Bis-(di-methoxyphosphonomethyl)-diphenyl (88 % Aktivsubstanz; 0,25 Mol) und 142,4 g Benzaldehyd-2-sulfonsäure Natriumsalz (80,4 % Aktivsubstanz; 0,55 Mol) vorgelegt. Der Autoklav wird geschlossen, auf 0°C gekühlt und bei dieser Temperatur 210 g flüssiges Ammoniak unter Rühren zudosiert, wobei eine gelbe Suspension entsteht. Zu dieser Suspension wird innerhalb 15 Minuten, die Natriumamid-Suspension vom ersten Glasautoklav unter Rühren zudosiert, wobei die Reaktionstemperatur von 0°C (4,1 bar) auf 9°C (5,7 bar) steigt und eine gelbe, kristalline Suspension des Reaktionsproduktes entsteht. Der erste Autoklav wird einmal mit 50 g flüssigem Ammoniak gespült und die Spüllösung in den zweiten Glasautoklaven zudosiert. Das Reaktionsgemisch wird nun eine Stunde bei 10°C weitergerührt und der Überschuss am Natriumamid schliesslich durch Zugabe von 5 g (0,125 Mol) gasförmiger Chlorwasserstoff neutralisiert.

Zur Aufarbeitung wird der Druck im zweiten Glasautoklaven durch partielle Verdampfung des Ammoniaks von 6,4 bar auf atmosphärischem Druck gesenkt, wobei die Innentemperatur von +10C auf -30°C sinkt, das Reaktionsgemisch mit 500 ml Wasser verdünnt und die erhaltene Suspension durch langsames Aufheizen auf +20°C weitgehend vom Ammoniak befreit. Der Rührer wird abgestellt und der Autoklav ausgeladen.

Das Reaktionsgemisch wird am Rotationsverdampfer unter Vakuum zur Trockene eingeengt, bei etwa 90°C mit einer Lösung von 81 g Natriumchlorid in 375 ml Wasser aufgenommen und auf Raumtemperatur abgekühlt. Das Reaktionsprodukt wird abgenutscht, mit 250 ml einer 7,5 %igen Natriumchlorid-Lösung gewaschen, unter Vakuum auf 100°C erhitzt und bis zur Gewichtskonstanz getrocknet.

Man erhält 141,7 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 93,2 % aufweist. Die Ausbeute an 4,4'-Bis-(2-sulfostyryl)-diphenyl Dinatriumsalz beträgt 93,9 % der Theorie.

### Beispiel 7:

Beispiel 6 wird wiederholt, wobei anstelle des 10 %igen Überschusses an Benzaldehyd-2-sulfonsäure Natriumsalz nur ein 7 %iger Überschuss, d.h. 138,5 g (80,4 % Aktivsubstanz; 0,535 Mol) verwendet wird.

Man erhält 148,4 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 90,9 % aufweist. Die Ausbeute an 4,4'-Bis-(2-sulfostyryl)-diphenyl Dinatriumsalz beträgt 95,9 % der Theorie.

### Beispiel 8:

Beispiel 6 wird wiederholt, wobei die zwei Ausgangsprodukte 4,4'-Bis-(dimethoxy-phosphonomethyl)-diphenyl und Benzaldehyd-2-sulfonsäure Natriumsalz in 260 g flüssigem Ammoniak anstelle von 210 g aufgenommen werden.

Man erhält 147,1 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 91,4 % aufweist. Die Ausbeute an 4,4'-Bis-(2-sulfostyryl)-diphenyl Dinatriumsalz beträgt 95,6 % der Theorie.

### Beispiel 9:

Eine Apparatur bestehend aus einem 1 l BUECHI®-Glasautoklaven, ausgerüstet mit Kühl/Heiz-Mantel, Manometer (0 bis 10 bar) und TESCOM®-Back-Pressure-Regulator (0 bis 7 bar), Rührer mit Permanent-Magnetantrieb, Thermometerhülse, Einlaufstutzen und Berstscheibe (10 bar), wird auf -45°C gekühlt.

In diesem Glasautoklaven werden 320 g flüssiges Ammoniak bei -45°C vorgelegt und unter Rühren, nach Zugabe von einem Stück von etwa 0,5 g Natrium und von 0,3 g Eisen(III)-nitrat Nonahydrat, innerhalb 20 Minuten insgesamt 5,8 g (0,25 Mol) Natrium in kleinen Stücken zugegeben, wobei aus dem Reaktionsgefäss ein Wasserstoffstrom entweicht. Die entstandene grau-schwarze Natriumamid-Suspension wird bei -45°C für 30 Minuten weitergerührt.

Zu dieser Natriumamid-Suspension werden bei -45°C bis -38°C innerhalb 5 Minuten 40,7 g 4,4'-Bis-(dimethoxyphosphonomethyl)-diphenyl (98 % Aktivsubstanz; 0,1 Mol) unter Rühren, mittels eines Pulverdosiertrichters, zudosiert, wobei eine dunkelrote Suspension entsteht, die für 30 Minuten bei -45°C weitergerührt wird. Zu dieser dunkelroten Suspension werden nun innerhalb von 5 Minuten, unter Rühren bei -45°C bis -33°C, 50,5 g Benzaldehyd-2-sulfonsäure Natriumsalz, (99 % Aktivsubstanz; 0,24 Mol) mittels eines Pulverdosiertrichters zudosiert.

Der Glasautoklav wird geschlossen und die dunkelrote Suspension bei -10°C (2 bar) für 4 Stunden weitergerührt, wobei die dunkelrote Farbe verschwindet und eine kristalline, hellgelbe Suspension des Reaktionsproduktes entsteht. Der Überschuss am Natriumamid wird dann durch Zugabe von 2 g (0,05 Mol) gasförmiger Chlorwasserstoff neutralisiert.

Zur Aufarbeitung wird der Druck im Glasautoklav durch partielle Verdampfung des Ammoniaks von 2 bar auf atmosphärischem Druck gesenkt, wobei die Innentemperatur von -10°C auf -33°C sinkt. Das Reaktionsgemisch wird mit 300 ml Wasser verdünnt und die erhaltene Suspension durch langsames Aufheizen auf +20°C weitgehend vom Ammoniak befreit. Der Rührer wird abgestellt und der Autoklav ausgeladen. Das Reaktionsgemisch wird schliesslich am Rotationsverdampfer unter Vakuum zur Trockene eingeengt, bei etwa 90°C mit einer Lösung von 30 g Natriumchlorid in 150 ml Wasser aufgenommen und auf Raumtemperatur abgekühlt. Das Reaktionsprodukt wird abgenutscht, mit 100 ml einer 7,5 %igen Natriumchlorid-Lösung gewaschen und unter Vakuum bei 100°C bis zur Gewichtskonstanz getrocknet.

Man erhält 52,5 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 91,4 % aufweist. Die Ausbeute an 4,4'-Bis-(2-sulfostyryl)-diphenyl Dinatriumsalz beträgt 85,3 % der Theorie.

### Beispiel 10:

Beispiel 9 wird wiederholt, wobei die Kondensation bei -35°C und unter atmosphärischem Druck durchgeführt wird.

Man erhält 54,1 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 89,2 % aufweist. Die Ausbeute an 4,4'-Bis-(2-sulfostyryl)-diphenyl Dinatriumsalz beträgt 85,8 % der Theorie.

### Beispiel 11:

In der unter Beispiel 9 beschriebenen Apparatur werden in der Reihenfolge,
67,8 g 4,4'-Bis-(dimethoxyphosphonomethyl)-diphenyl (88.1 % Aktivsubstanz; 0,15 Mol),
90,6 g Benzaldehyd-2-sulfonsäure Natriumsalz (79,3 % Aktivsubstanz; 0,345 Mol) und
230 g flüssiges Ammoniak
vorgelegt und bei 9°C (6 bar) gerührt.

Zu dieser blassgelben Suspension werden innerhalb von 10 Minuten 81,0 g einer methanolischen 30 %igen Natriummethylat-Lösung (0,45 Mol) unter Rühren zudosiert, wobei die Reaktionstemperatur von 9°C (6 bar) auf 20°C (6,6 bar) steigt und eine kristalline gelbe Suspension des Reaktionsproduktes entsteht. Das Reaktionsgemisch wird für eine Stunde bei 20°C weitergerührt und der Überschuss an Natriummethylat danach durch Zugabe von 6 g (0,15 Mol) gasförmigem Chlorwasserstoff neutralisiert.

Zur Aufarbeitung senkt man den Druck im Glasautoklav durch partielle Verdampfung des Ammoniaks von 6,6 bar auf atmosphärischem Druck, wobei die Innentemperatur von 20°C auf -19°C sinkt. Das Reaktionsgemisch wird mit 300 ml Wasser verdünnt und die erhaltene Suspension, durch langsames Aufheizen auf +20°C, weitgehend vom Ammoniak befreit. Der Rührer wird abgestellt und der Autoklav ausgeladen. Das Reaktionsgemisch wird schliesslich am Rotationsverdampfer unter Vakuum zum Trockene eingeengt, bei etwa 90°C mit einer Lösung von 45 g Natriumchlorid in 225 ml Wasser aufgenommen und auf Raumtemperatur abgekühlt. Das Reaktionsprodukt wird abgenutscht, mit 150 ml einer 7,5 %igen Natriumchlorid-Lösung gewaschen, unter Vakuum auf 100°C erhitzt und bis zur Gewichtskonstanz getrocknet.
Man erhält 83,6 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 94,0 % aufweist. Die Ausbeute an 4,4'-Bis-(2-sulfostyryl)-diphenyl Dinatriumsalz beträgt 93,1 % der Theorie.

### Beispiel 12:

Beispiel 11 wird wiederholt, wobei anstelle des 15 %igen Überschusses an Benzaldehyd-2-sulfonsäure Natriumsalz, nur ein 10 %iger Überschuss, d.h. 86,6 g (79,3 % Aktivsubstanz; 0,33 Mol) verwendet wird.

Man erhält 83,2 g 4,4'-Bis-(2-sulfostyryl)-diphenyl Dinatriumsalz in Form eines hellgelben kristallinen Pulvers mit einem Schmelzpunkt von über 300°C, das einen Gehalt an Aktivsubstanz (UV-spektralphotometrisch bestimmt) von 94,7 % aufweist. Die Ausbeute an 4,4'-Bis-(2-sulfostyryl)-diphenyl Dinatriumsalz beträgt 93,4 % der Theorie.

## Patentansprüche

1. Verfahren zur Herstellung von Distyrylbiphenylverbindungen der Formel (1) 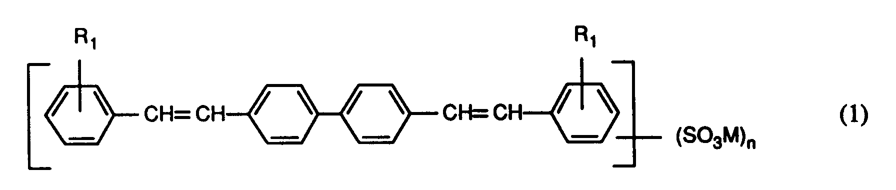 durch Kondensation einer Verbindung der Formel (2) 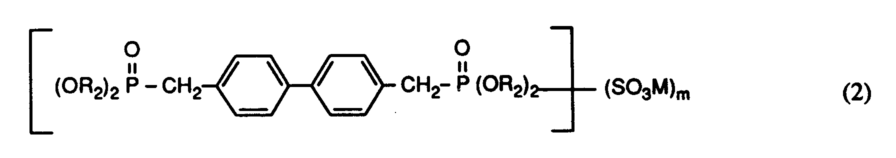 mit einer Verbindung der Formel (3) 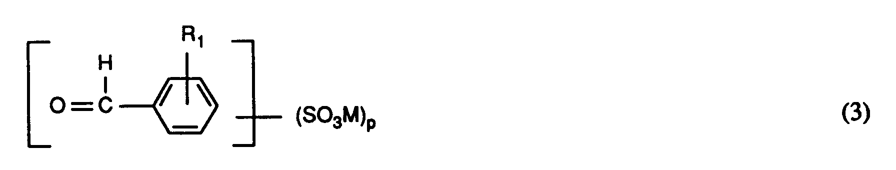 worin
R₁ Wasserstoff, C₁-C₅-Alkyl oder Halogen;
R₂ C₁-C₈-Alkyl;
M ein salzbildendes farbloses Kation;
n 2 oder 4;
m 0 oder 2; und
p 0 oder 1; unter der Voraussetzung, dass m + p 2 oder 4 ist,
bedeuten,
dadurch gekennzeichnet, das man die Kondensation in flüssigem Ammoniak in Gegenwart von stark alkalisch reagierenden Substanzen durchführt.

2. Verfahren gemäss Anspruch 1, dadurch gekennzeichnet, dass man eine Verbindung der Formel (4) 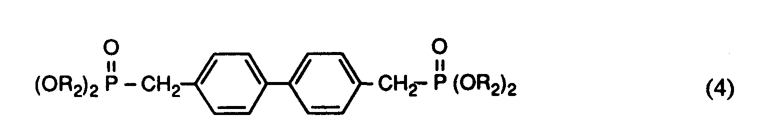 mit einer Verbindung der Formel (5) 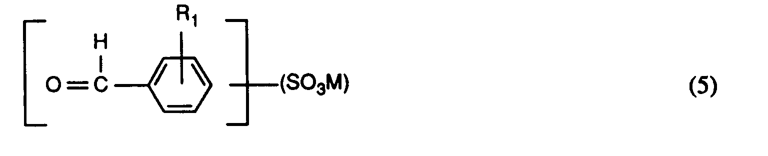 kondensiert, worin
R₁ Wasserstoff, C₁-C₅-Alkyl oder Halogen; R₂ C₁-C₈-Alkyl; und
M ein salzbildendes farbloses Kation
bedeutet.

3. Verfahren gemäss Anspruch 2, dadurch gekennzeichnet, dass man eine Verbindung der Formel (4) mit einer Verbindung der Formel (6) 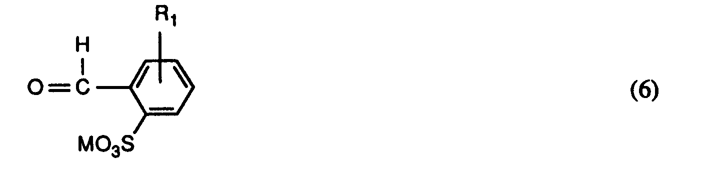 kondensiert, worin
R₁ Hydrogen, C₁-C₅-Alkyl oder Chlor;
R₂ C₁-C₈-Alkyl; und
M ein salzbildendes farbloses Kation
bedeutet.

4. Verfahren gemäss einem der Ansprüche 1 bis 3, dadurch gekennzeichnet, dass man als stark alkalisch reagierende Substanzen Alkalimetalle oder deren stark basische Verbindungen verwendet.

5. Verfahren gemäss Anspruch 4, dadurch gekennzeichnet, dass man als stark basische Verbindungen Amide, Hydride oder Alkoholate von Alkalimetallen oder Gemische davon verwendet.

6. Verfahren gemäss Anspruch 5, dadurch gekennzeichnet, dass man als stark basische Verbindungen Alkoholate oder Amide des Natriums verwendet.

7. Verfahren gemäss Anspruch 6, dadurch gekennzeichnet, dass man als stark basische Verbindung Natriumamid verwendet.

8. Verfahren gemäss Anspruch 7, dadurch gekennzeichnet, dass man das verwendete Natriumamid durch Lösen von Natrium in flüssigem Ammoniak in Gegenwart eines Katalysators herstellt.

9. Verfahren gemäss einem der Ansprüche 1 bis 8, dadurch gekennzeichnet, dass man die Kondensation bei Temperaturen zwischen -40°C und 25°C durchführt.

10. Verfahren gemäss Anspruch 9, dadurch gekennzeichnet, dass man die Kondensation bei Temperaturen zwischen 0°C und 25°C durchführt.

11. Verfahren gemäss Anspruch 10, dadurch gekennzeichnet, dass man die Kondensation bei Temperaturen zwischen 10°C und 20°C durchführt.

12. Verfahren gemäss einem der Ansprüche 1 bis 11, dadurch gekennzeichnet, dass die Verbindung der Formel (1) durch Filtration aus dem flüssigen Ammoniak von den Nebenprodukten abgetrennt wird.

13. Verfahren gemäss einem der Ansprüche 1 bis 12, dadurch gekennzeichnet, dass der Ammoniak recycliert wird.

14. Verfahren gemäss Anspruch 1, dadurch gekennzeichnet, dass das Verhältnis der Verbindungen der Formeln (2) und (3) 1:2 bis 1:2,5 beträgt.

15. Verfahren gemäss Anspruch 14, dadurch gekennzeichnet, dass das Verhältnis der Verbindungen der Formeln (2) und (3) 1:2,1 bis 1:2,2 beträgt.

16. Verfahren gemäss Anspruch 1 zur Herstellung der Verbindungen der Formel (7) 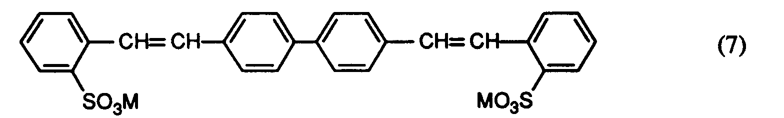 durch Kondensation einer Verbindung der Formel (4) 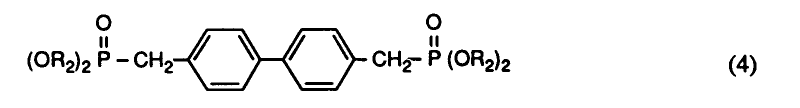 mit einer Verbindung der Formel (8) 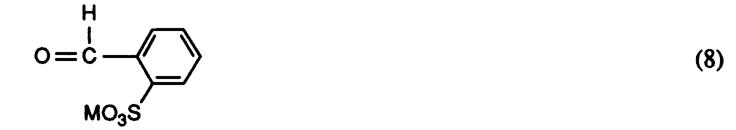 worin R₂ C₁-C₈-Alkyl und M ein Natrium-, Kalium- oder Ammoniumion bedeuten, dadurch gekennzeichnet, dass man
a) flüssiges Ammoniak vorlegt;
b) Natrium und einen Katalysator zugibt;
c) die so erhaltene Natriumamid-Suspension zu einer Suspension der Verbindungen der Formel (4) und (8) in flüssigem Ammoniak zudosiert;
d) das überschüssige Natriumamid neutralisiert;
e) das Endprodukt der Formel (7) abfiltriert; und
f) den Ammoniak durch Abdampfen und erneutes Kondensieren wiedergewinnt.

## Claims

1. A process for preparing distyrylbiphenyl compounds of the formula (1) 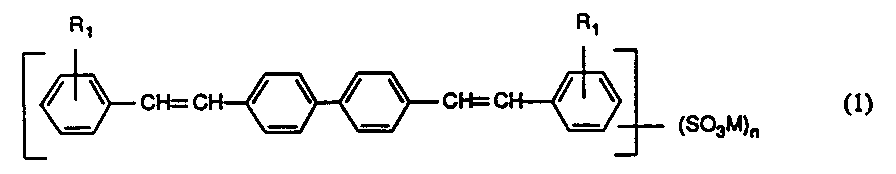 by condensation of a compound of the formula (2) 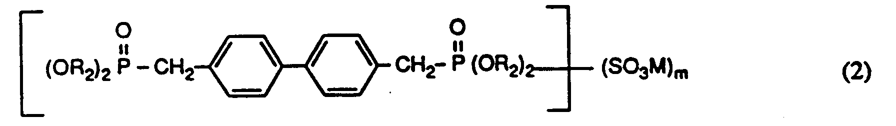 with a compound of the formula (3) 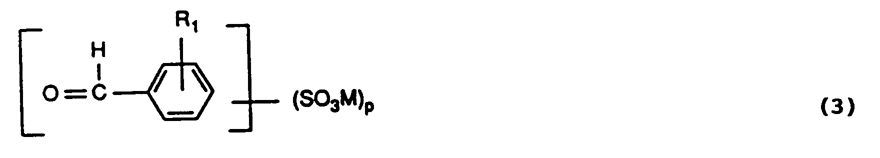 in which
R₁ is hydrogen, C₁-C₅alkyl or halogen;
R₂ is C₁-C₈alkyl;
M is a salt-forming colourless cation;
n is 2 or 4;
m is 0 or 2; and
p is 0 or 1; provided m + p is 2 or 4,
which comprises carrying out the condensation in liquid ammonia in the presence of strongly alkaline substances.

2. A process according to Claim 1, wherein a compound of the formula (4) 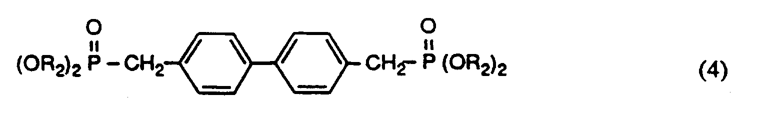 is condensed with a compound of the formula (5) 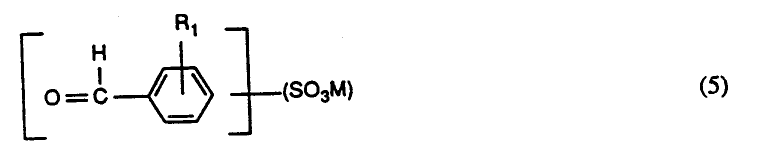 in which
R₁ is hydrogen, C₁-C₅alkyl or halogen; R₂ is C₁-C₈alkyl; and
M is a salt-forming colourless cation.

3. A process according to Claim 2, wherein a compound of the formula (4) is condensed with a compound of the formula (6) 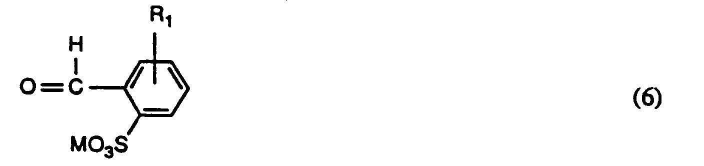 in which
R₁ is hydrogen, C₁-C₅alkyl or chlorine;
R₂ is C₁-C₈alkyl; and
M is a salt-forming colourless cation.

4. A process according to any one of Claims 1 to 3, wherein alkali metals or strongly basic compounds thereof are used as the strongly alkaline substances.

5. A process according to Claim 4, wherein amides, hydrides or alcoholates of alkali metals or mixtures thereof are used as the strongly basic compounds.

6. A process according to Claim 5, wherein sodium alcoholates or sodium amides are used as the strongly basic compounds.

7. A process according to Claim 6, wherein sodium amide is used as the strongly basic compound.

8. A process according to Claim 7, wherein the sodium amide used is prepared by dissolving sodium in liquid ammonia in the presence of a catalyst.

9. A process according to any one of Claims 1 to 8, wherein the condensation is carried out at temperatures of between -40°C and 25°C.

10. A process according to Claim 9, wherein the condensation is carried out at temperatures of between 0°C and 25°C.

11. A process according to Claim 10, wherein the condensation is carried out at temperatures of between 10°C and 20°C.

12. A process according to any one of Claims 1 to 11, wherein the compound of the formula (1) is separated off from the byproducts by filtration from the liquid ammonia.

13. A process according to any one of Claims 1 to 12, wherein the ammonia is recycled.

14. A process according to Claim 1, wherein the ratio of the compounds of the formulae (2) and (3) is 1:2 to 1:2.5.

15. A process according to Claim 14, wherein the ratio of the compounds of the formulae (2) and (3) is 1:2.1 to 1:2.2.

16. A process according to Claim 1 for preparing the compounds of the formula (7) 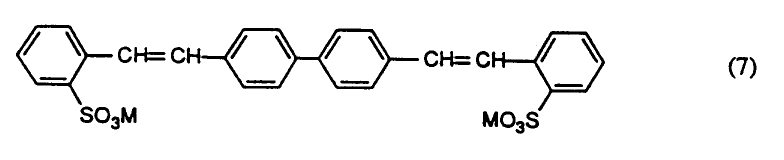 by condensation of a compound of the formula (4) 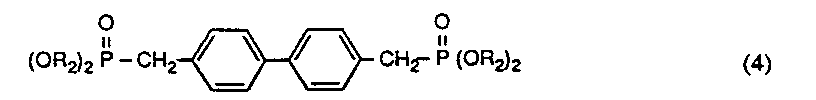 with a compound of the formula (8) 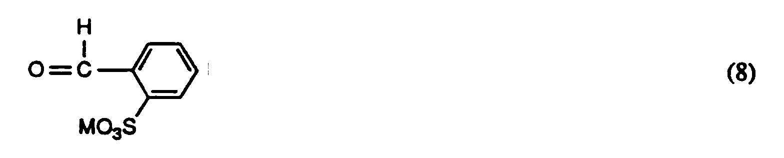 in which R₂ is C₁-C₈alkyl and M is a sodium ion, potassium ion or ammonium ion, wherein
a) liquid ammonia is introduced as the initial charge;
b) sodium and a catalyst are added;
c) the sodium amide suspension thus obtained is metered into a suspension of the compounds of the formula (4) and (8) in liquid ammonia;
d) the excess sodium amide is neutralized;
e) the end product of the formula (7) is filtered off; and
f) the ammonia is recovered by evaporation and recondensation.

## Revendications

1. Procédé de préparation de composés de type distyrylbiphényle, de formule (1) 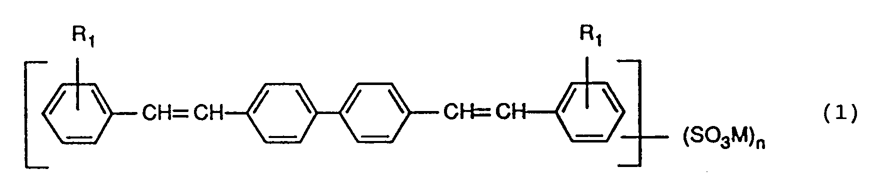 par condensation d'un composé de formule (2) 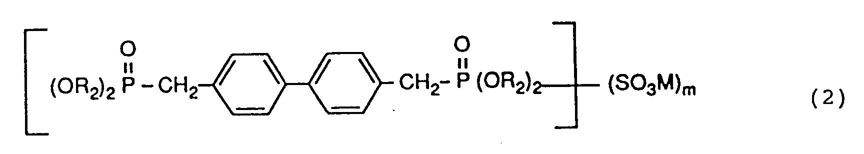 avec un composé de formule (3) 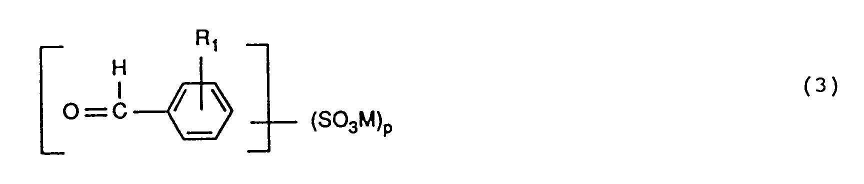 dans lesquelles formules
R₁ représente un atome d'hydrogène ou d'halogène ou un groupe alkyle en C₁₋₅,
R₂ représente un groupe alkyle en C₁₋₈,
M représente un cation incolore formant un sel,
n vaut 2 ou 4,
m vaut 0 ou 2, et
p vaut 0 ou 1,
à condition que (m + 2p) vaille 2 ou 4,
**caractérisé** en ce que l'on effectue cette condensation dans de l'ammoniac liquide et en présence d'une substance à forte réaction alcaline.

2. Procédé conforme à la revendication 1, **caractérisé** en ce que l'on condense un composé de formule (4) 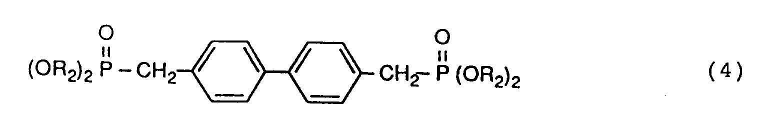 avec un composé de formule (5) 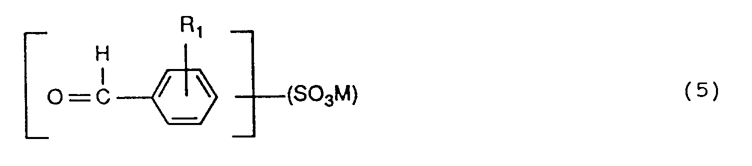 dans lesquelles formules R₁ représente un atome d'hydrogène ou d'halogène ou un groupe alkyle en C₁₋₅, R₂ représente un groupe alkyle en C₁₋₈ et M représente un cation incolore formant un sel.

3. Procédé conforme à la revendication 2, **caractérisé** en ce que l'on condense un composé de formule (4) avec un composé de formule (6) 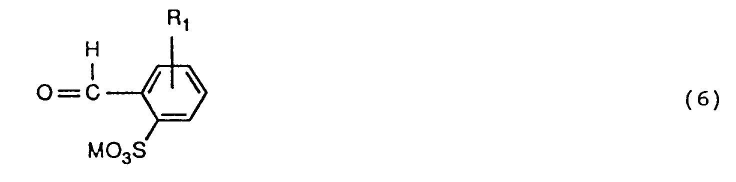 où R₁ représente un atome d'hydrogène ou de chlore ou un groupe alkyle en C₁₋₅, R₂ représente un groupe alkyle en C₁₋₈ et M représente un cation incolore formant un sel.

4. Procédé conforme à l'une des revendications 1 à 3, **caractérisé** en ce que l'on utilise, en tant que substance à forte réaction alcaline, un métal alcalin ou un composé fortement basique d'un tel métal.

5. Procédé conforme à la revendication 4, **caractérisé** en ce que l'on utilise, en tant que composés fortement basiques, des amides, hydrures ou alcoolates de métaux alcalins, ou des mélanges de tels composés.

6. Procédé conforme à la revendication 5, **caractérisé** en ce que l'on utilise, en tant que composé fortement basique, de l'amidure de sodium ou un alcoolate de sodium.

7. Procédé conforme à la revendication 6, **caractérisé** en ce que l'on utilise, comme composé fortement basique, de l'amidure de sodium.

8. Procédé conforme à la revendication 7, **caractérisé** en ce que l'on prépare l'amidure de sodium utilisé en dissolvant du sodium dans de l'ammoniac liquide, en présence d'un catalyseur.

9. Procédé conforme à l'une des revendications 1 à 8, **caractérisé** en ce que l'on effectue la condensation à une température située entre -40°C et 25°C.

10. Procédé conforme à la revendication 9, **caractérisé** en ce que l'on effectue la condensation à une température située entre 0°C et 25°C.

11. Procédé conforme à la revendication 10, **caractérisé** en ce que l'on effectue la condensation à une température située entre 10°C et 20°C.

12. Procédé conforme à l'une des revendications 1 à 11, **caractérisé** en ce que l'on sépare le composé de formule (1) par filtration, dans l'ammoniac liquide, d'avec les produits secondaires.

13. Procédé conforme à l'une des revendications 1 à 12, **caractérisé** en ce que l'ammoniac est recyclé.

14. Procédé conforme à la revendication 1, **caractérisé** en ce que le rapport entre les composés de formules (2) et (3) vaut de 1/2 à 1/2,5.

15. Procédé conforme à la revendication 1, **caractérisé** en ce que le rapport entre les composés de formules (2) et (3) vaut de 1/2,1 à 1/2,2.

16. Procédé, conforme à la revendication 1, de préparation de composés de formule (7) 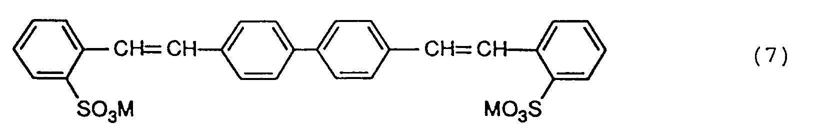 par condensation d'un composé de formule (4) 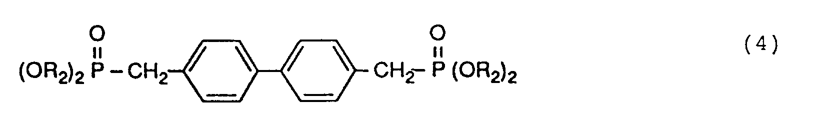 et d'un composé de formule (8) 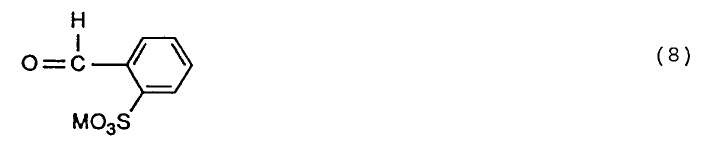 dans lesquelles formules R₂ représente un groupe alkyle en C₁₋₈ et M représente un ion sodium, potassium, ou ammonium, **caractérisé** en ce que :
a) on prend de l'ammoniac liquide ;
b) on y ajoute du sodium et un catalyseur ;
c) on ajoute la suspension d'amidure de sodium ainsi obtenue à une suspension des composés de formules (4) et (8) ;
d) on neutralise l'excès d'amidure de sodium ;
e) on recueille par filtration le produit final, de formule (7) ; et
f) on en chasse l'ammoniac par évaporation, et on récupère celui-ci en le condensant à nouveau.
